# EUROPÄISCHE PATENTSCHRIFT

(11) **EP 0 631 497 B1**
(45) Veröffentlichungstag und Bekanntmachung des Hinweises auf die Patenterteilung: **05.03.1997**
(21) Anmeldenummer: 93901615.0
(22) Anmeldetag: 19.01.1993
(51) Int. Cl.: A61F 2/30

(54) **SCHAFT FÜR EINE GELENKENDOPROTHESE**
SHAFT FOR AN ARTICULATION ENDOPROSTHESIS
TIGE POUR ENDOPROTHESE ARTICULAIRE

(43) Veröffentlichungstag der Anmeldung: 04.01.1995
(73) Patentinhaber: MATHYS AG BETTLACH CHIRURGISCHE INSTRUMENTE UND IMPLANTATE, CH-2544 Bettlach (CH)
(72) Erfinder: MATHYS, Robert, Sen., CH-2544 Bettlach (CH); MATHYS, Robert, Jr., CH-2544 Bettlach (CH); GASSER, Beat, CH-3063 Ittigen (CH)
(74) Vertreter: Lusuardi, Werther Giovanni, Dr.
(86) Internationale Anmeldenummer: CH9300008
(87) Internationale Veröffentlichungsnummer: WO9416649

(56) Entgegenhaltungen:
- EP-A- 0 484 082
- WO-A-86/06617
- DE-A- 2 933 237
- DE-A- 3 525 547
- DE-A- 3 840 475
- FR-A- 2 199 967
- GB-A- 2 045 082
- US-A- 4 454 612

## Beschreibung

Die Erfindung bezieht sich auf einen Schaft für eine Gelenkendoprothese, gemäss der Gattung des Patentanspruchs 1 und eine Gelenkendoprothesenkomponente mit einem solchen Schaft, gemäss Anspruch 27.

Aus der EP-A-0484082 ELDON ist eine gettungsgemäße Femurkomponente für eine Hüftendoprothese bekannt, welche eine poröse und damit relativ dicke Oberflächenschicht aufweist.

Gelenkendoprothesen, insbesondere solche für das Hüftgelenk bestehen aus einer schalenförmigen Acetabularkomponente und einer Femurkomponente, welche mittels ihres Schaftes in das röhrenförmige Femur eingesetzt werden kann.

Es sind bereits Femurkomponenten für Hüftprothesen bekannt, welche im wesentlichen aus einem metallischen Kern und einem diesen umgebenden Kunststoffmantel bestehen. Diese Konstruktionen besitzen den Vorteil, dass sie sich im Vergleich zur zu ersetzenden Knochenstruktur annähernd "gleich-elastisch" verhalten. Fraglich bei dieser Art von Femurkomponenten ist die ungeschützte Polymeroberfläche des Mantels, aus welcher Restmonomere freigesetzt werden und zur Ausbildung einer dünnen Bindegewebsschicht zwischen dem Kunststoffmantel und dem Knochen führen können.

Hier will die Erfindung Abhilfe schaffen. Der Erfindung liegt die Aufgabe zugrunde, einen Schaft für eine Gelenkendoprothese zu schaffen, der einerseits ein verbessertes elastisches, den physiologischen Bedürfnissen des Knochens angepasstes Verhalten aufweist, derart dass die Flexibilität des Schaftes unter einer Biegebelastung annähernd derjenigen des natürlichen Knochens entspricht sowie einen graduellen Lasttransfer vom Schaft zum umgebenden Knochen ermöglicht und anderseits eine verbesserte Biokompatibilität gewährt. Die Erfindung löst weiter die Aufgabe einen Schaft für eine Gelenkendoprothese mit einer verbesserten Oberflächenhärte zu schaffen.

Die Erfindung löst die gestellte Aufgabe mit einem Schaft für eine Gelenkendoprothese, welcher die Merkmale des Anspruchs 1 aufweist und einer Gelenkendoprothesenkomponente mit einem solchen Schaft, welche die Merkmale des Anspruchs 28 aufweist.

Weitere vorteilhafte Ausgestaltungen der Erfindung sind in den abhängigen Ansprüchen gekennzeichnet.

Die durch die Erfindung erreichten Vorteile sind im wesentlichen darin zu sehen, dass dank der erfindungsgemässen Oberflächenschicht die Biokompatibilität des Schaftes insgesamt verbessert wird und ein direkter und fester Schaft/Knochen-Kontakt ermöglicht wird. Je nach Art dieser biokompatiblen Oberflächenschicht kann das Knochenwachstum an den Knochen induziert werden, was die Primärstabilität, d.h. die anfängliche Verankerung verbessert und damit die Einheilungsphase beschleunigt.

Bei einer bevorzugten Ausführungsform der Erfindung ist der Schaft im proximo-lateralen Bereich, d.h. an der äusseren Seite des oberen Schaftendes mit einer oder mehreren Bohrungen versehen. Diese Bohrungen dienen der Verankerung des Prothesenschaftes im proximalen Bereich der äusseren Kortikalis mittels geeigneter Schrauben. Dabei kann diese Schraube von oben, d.h. von der Seite des Prothesenhalses, also vom Prothesenkragen, durch die Bohrung hindurch lateral im Knochen eingeschraubt und dort verankert werden. Es ist aber auch möglich, die Bohrung mit einem Innengewinde zu versehen und die Schraube von unten, d.h. lateral in das Innengewinde einzuschrauben, wobei die Auflagefläche des Schraubenkopfes an der äusseren Kortikalis mittels einer Unterlagsscheibe vergrössert werden kann. Diese Schrauben verbessern einerseits die Rotationsstabilität des Schaftes innerhalb des Markkanals und tragen andererseits in synergistischer Weise mit dem erfindungsgemässen dreischichtigen Aufbau des Prothesenschaftes zu einem graduellen Lasttransfer vom Schaft zum Knochen bei.

Die Erfindung und Weiterbildungen der Erfindung werden im folgenden anhand eines Ausführungsbeispiels noch näher erläutert:
Fig. 1 zeigt einen Längsschnitt durch einen im Oberschenkelknochen eingesetzten erfindungsgemässen Schaft einer Hüftgelenk-Femurkomponente;
Fig. 2 zeigt einen Querschnitt längs der Linie A-A im proximalen Teil des Schaftes nach Fig. 1;
Fig. 3 zeigt einen Querschnitt längs der Linie B-B im distalen Teil des Schaftes nach Fig. 1;
Fig. 4 zeigt in einer Prinzipskizze den schematischen Aufbau der Oberflächenschicht des Schaftes nach Fig. 1 am Beispiel einer Partikelbeschichtung bei materialmässig einheitlicher Zusammensetzung dieser Oberflächenschicht; und
Fig. 5 zeigt in einer Prinzipskizze den schematischen Aufbau der Oberflächenschicht des Schaftes nach Fig. 1 am Beispiel einer Partikelbeschichtung bei einem Verbundaufbau dieser Oberflächenschicht.

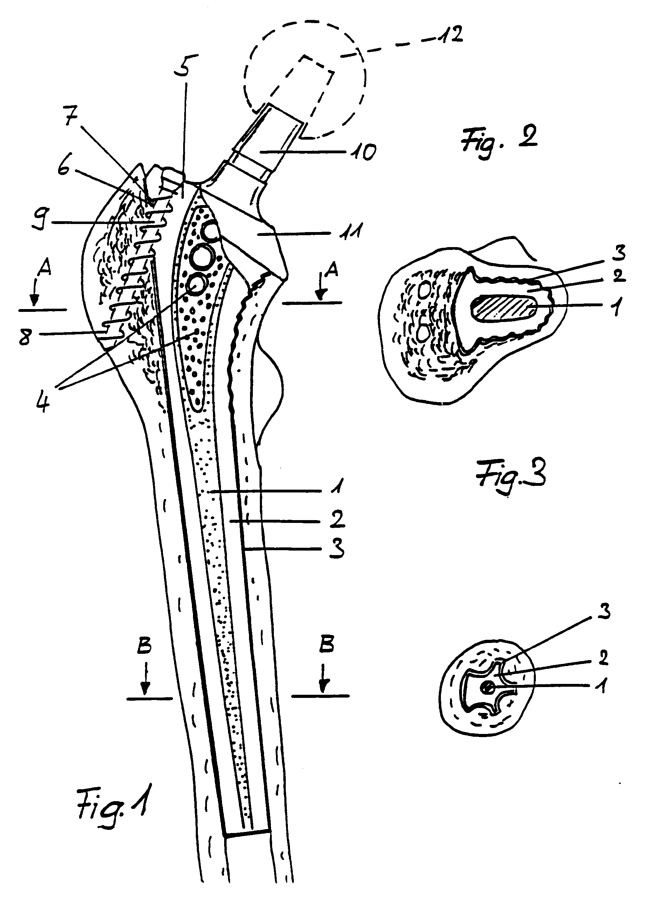

Die in den Fig. 1 - 3 dargestellte Femurkomponente einer Hüftgelenkprothese besteht im wesentlichen aus dem erfindungsgemässen, dreischichtig aufgebauten Schaft 1,2,3 der sich in einer konischen Halspartie 10 fortsetzt, auf welche eine Gelenkkugel 12 aufsteckbar ist. Bei anderen Hüftgelenkprothesentypen kann die Gelenkkugel auch fest mit der Halspartie 10 verbunden sein und mit dem Schaft ein Ganzes bilden.

Der sandwichartig aufgebaute Schaft 1,2,3 besteht aus einem metallischen Kern 1 aus Titan oder einer Titanlegierung, der in einem Mantel 2 aus biokompatiblem, thermoplastischem Kunststoff (z.B. POM, PEEK, PEI oder ähnliche) eingebettet ist. Die beiden Materialien Metall/Kunststoff sind deutlich abgegrenzt und werden form- und/oder kraftschlüssig zusammengehalten. Dies wird durch die im Kern 1 angebrachten kleineren (nicht-durchgängigen) und grösseren (durchgängigen) Löcher 4 verbessert, in welche sich entsprechende zapfenförmige Fortsätze des Mantels 2 erstrecken. Fabrikatorisch kann das Aufbringen des Mantels 2 auf den Kern 1 durch Spritzgiessen, Kleben und/oder nachträgliche mechanische Bearbeitung des Kunststoffmantels erfolgen.

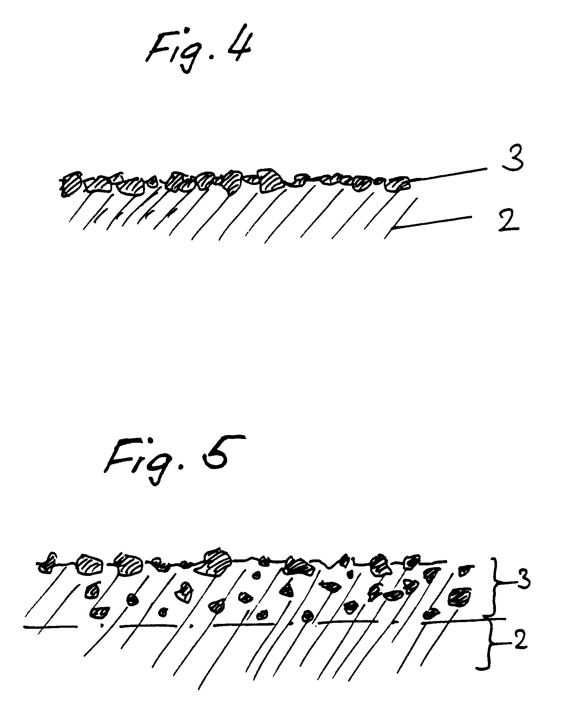

Umfangreiche Versuche haben ergeben, dass durch optimale Wahl des Verhältnis Vₖ/Vₘ der Volumina des Kerns 1 und des Mantels 2 besonders gute Resultate bezüglich des elastischen, den lokalen, physiologischen Bedürfnissen des Knochens angepassten Verhaltens des Schaftes erzielbar sind. Das Verhältnis Vₖ/Vₘ sollte deshalb vorteilhafterweise im Bereich von 0,15 - 0,60, vorzugsweise zwischen 0,25 - 0,4 liegen. Wie aus einem Vergleich der beiden Querschnitte im proximalen Abschnitt des Schaftes (Fig. 2) und im distalen Abschnitt des Schaftes (Fig. 3) resultiert, verändert sich das Verhältnis der Volumina in diesen beiden Abschnitten. Das Verhältnis der Volumina des Kerns 1 und des Mantels 2 im distalen Bereich des Schaftes, welcher 2/3 der Gesamtlänge des Schaftes beträgt, sollte im Bereich von 0,10 - 0,45, vorzugsweise zwischen 0,2 - 0,3 liegen. Der Kern kann innerhalb des Prothesenschaftes sowohl zentrisch als auch exzentrisch zu liegen kommen, d.h. die Mittelachse von Kern und Mantel müssen nicht unbedingt zusammenfallen. Eine exzentrische Lage des Kerns kann, je nach Aufbau des Prothesenschaftes, für eine optimale, graduelle Lastverteilung, respektive Lasttransfer, von Vorteil sein.

Der Mantel 2 aus Kunststoff ist seinerseits von einer relativ dünnen Oberflächenschicht 3 (max. 600 µm, vorzugsweise weniger als 200 µm dick) umgeben, welche aus einem biokompatiblen Material besteht und an ihrer äusseren Seite eine komplexe Strukturierung aufweist. Vorzugsweise wird die Oberflächenschicht derart ausgebildet, dass sie keine innere Porenstruktur aufweist.

Im proximalen Bereich des Schaftes, der 1/3 der Gesamtlänge des Schaftes beträgt (von der Halspartie 10 in distaler Richtung an gemessen) ist im Mantel 2 aus Kunststoff eine Makrostrukturierung mit einer Rauhtiefe zwischen 0,2 - 2,5 mm, vorzugsweise zwischen 0,5 - 1,5 mm vorhanden. Im distalen Bereich des Schaftes, der 2/3 der Gesamtlänge des Schaftes beträgt (von der Schaftspitze in Richtung proximal gemessen), ist eine Mikrostrukturierung mit einer Rauhtiefe zwischen 0,1 - 500,0 µm, vorzugsweise zwischen 5 - 80 µm vorhanden. Im proximalen Bereich des Schaftes kann zusätzlich zur Makrostrukturierung durch die Oberflächenschicht auch eine Mikrostrukturierung mit einer Rauhtiefe zwischen 0,1 - 500,0 µm, vorzugsweise zwischen 5 - 300 µm vorhanden sein. Besonders vorteilhaft ist eine Rauhtiefe zwischen 5 - 100 µm oder zwischen 150 - 300 µm zur Verstärkung der Oberflächenrauhigkeit.

Als biokompatible Materialien für den Kern 1 des erfindungsgemässen Schaft kommen Reintitan, Titanlegierungen, insbesondere aus TiAl₆V₄, TiAl₅Fe_{2.5} oder TiAl₆Nb₇ in Frage. Für den Mantel 2 des erfindungsgemässen Schaft eignen sich Polymere, wie z.B. Polyoxymethylen (POM), Polyether-Etherketon (PEEK), Polyaryletherketon (PAEK), Polyetherimid (PEI), Polymethylpenten (PMP), Polysulfon (PSU), Polyäthersulfon (PESU oder PES), Polyäthylenterephthalat (PETP), Polymethylmethacrylat (PMMA), Ultrahochmolekulares-Polyäthylen (UHMW-PE) oder flüssigkristallines Polymer (LCP).
Für die Oberflächenschicht kommen Biogläser, Calciumphosphate, insbesondere Hydroxylapatit, Kombinationen davon, Reintitan, Titanlegierungen, insbesondere aus TiAl₆V₄, TiAl₅Fe_{2.5} oder TiAl₆Nb₇ oder Materialkombinationen aus einem dieser Materialien und dem Material des Mantels aus Kunststoff als Matrix in Betracht.
Die Materialien Kunststoff/Oberflächenschicht der beiden Strukturen (Mantel 2 und Oberflächenschicht 3) sind deutlich voneinander abgegrenzt, d.h. sie weisen keine ausgeprägte Übergangszone (Durchmischung) auf. Auch wenn Materialpartikel der Oberflächenschicht 3 fertigungstechnisch in den Mantel 2 aus Kunststoff (Matrix) eingelagert sind, ist die Dicke dieser Oberflächenverbundschicht klar definierbar (siehe Fig. 5), indem sie aus derjenigen Schicht besteht, welche solche Materialpartikel enthält, währenddem der Mantel 2 aus reinem, partikelfreiem Kunststoff besteht.
Die Oberflächenschicht 3 kann in ihrer materialmässigen Zusammensetzung einheitlich sein oder selbst wieder aus mehreren Materialien zusammengesetzt sein. Im Verhältnis zu den beiden anderen Strukturen (Kern 1 und Mantel 2) ist die Oberflächenschicht 3 relativ dünn dimensioniert, so dass die mechanischen Eigenschaften des Schaftes durch die Oberflächenschicht 3 insgesamt nicht verändert werden. Bei materialmässig einheitlicher Zusammensetzung dieser Oberflächenschicht 3 ist diese einlagig, wie in Fig. 4, am Beispiel einer Partikelbeschichtung, dargestellt. Es besteht, in diesem Fall, auch kein Zusammenhalt zwischen den einzelnen Partikeln der Oberflächenschicht, diese sind also einzig mit dem Substrat, d.h. mit dem aus Kunststoff bestehenden Mantel 2 verbunden. Bei materialmässig durchmischter Zusammensetzung dieser Oberflächenschicht 3 kann diese als mehrlagig bezeichnet werden, wie in Fig. 5, am Beispiel einer Partikelbeschichtung bei Verbundaufbau, dargestellt. Auch bei dieser Variante der Oberflächenschicht 3 besteht der Zusammenhalt zwischen den einzelnen Partikeln einzig durch die Matrix aus Kunststoff, welche vorzugsweise aus dem gleichen Material wie der Mantel 2 besteht.

Das Aufbringen der Oberflächenschicht 3 auf den Mantel 2 kann mittels verschiedener bekannter Verfahren, wie z.B. Bedampfung (Physical, Chemical Vapour Deposition PVD/CVD), Kalt-, Heiss-Pressen, Flammspritzen, Plasmaspritzen, Laserbehandlung, Sintern, Schrumpfen, Spritzgiessen, Diffusions-, Ultraschall-Beschichten oder ähnliches erfolgen.

Im proximo-lateralen Bereich 5 des erfindungsgemässen Schaftes ist eine Bohrung 6 vorgesehen, durch welche - wie in Figur 1 dargestellt - eine Schraube 9 in der äusseren Kortikalis 8 des Oberschenkelknochens verankert ist. Die Schraube 9 kann als Zugschraube mit langem Schaftteil oder als Schraube mit Vollgewinde ausgebildet sein. Bei einer Zugschraube, wie bei einer Schraube mit Vollgewinde, entsteht eine kraftschlüssige Verbindung zwischen Prothesenkragen 11 und dem Oberschenkelknochen. Bei einer Schraube mit Vollgewinde, das im Prothesenkragen und im Knochen verankert ist, entsteht eine mechanisch stabilere Verbindung zwischen Prothese und Knochen, weil auch Bewegungen zwischen Schraube 9 und Knochen sowie Schraube 9 und Prothesenkragen 11 verhindert werden. Die Schraube 9 kann auch umgekehrt von unten, d.h. lateral in das Innengewinde 7 der Bohrung 6 eingeschraubt werden, wobei dann zweckmässigerweise die Auflagefläche des Schraubenkopfes an der äusseren Kortikalis mittels einer Unterlagsscheibe vergrössert wird.

Der Kern 1 tritt nach oben aus dem Mantel 2 heraus und bildet eine konische Halspartie 10, auf welche Gelenkkugeln 12 verschiedener Grössen - in bekannter Weise - mit entsprechenden Innenkonen aufgesteckt werden können.

Der für eine Anwendung als Hüftgelenkersatz beschriebene erfindungsgemässe Schaft kann auch beim Ersatz anderer Gelenke, z.B. Schulter- oder Fingergelenke, verwendet werden.

## Patentansprüche

1. Schaft für eine Gelenkendoprothese mit einem metallischen Kern (1) und einer proximal daran anschliessenden Halspartie (10), sowie einem den Kern (1) distal umgebenden Mantel (2) aus einem nichtmetallischen, elastischen Material, wobei der Zusammenhalt zwischen Kern (1) und Mantel (2) kraft- und/oder formschlüssig ist, der Mantel (2) mit einer zusätzlichen Oberflächenschicht (3) aus biokompatiblem Material versehen ist und die Oberflächenschicht (3) eine höhere Oberflächenhärte aufweist als der Mantel (2), dadurch gekennzeichnet, dass
a) das nichtmetallische Material des Mantels (2) einen E-Modul zwischen 500 bis 10'000 N/mm² aufweist;
b) die Oberflächenschicht (3) eine Dicke von weniger als 600 µm aufweist;
c) die Oberflächenschicht (3) im Inneren porenfrei ausgebildet ist;
d) die Oberflächenschicht (3) an ihrer äusseren Seite im proximalen Bereich des Schaftes, der 1/3 der Gesamtlänge des Schaftes beträgt, eine Makrostrukturierung mit einer Rauhtiefe zwischen 0,2 - 2,5 mm aufweist; und
e) die Oberflächenschicht (3) an ihrer äusseren Seite im distalen Bereich des Schaftes, der 2/3 der Gesamtlänge des Schaftes beträgt, eine Mikrostrukturierung mit einer Rauhtiefe zwischen 0,1 - 500,0 µm aufweist.

2. Schaft nach Anspruch 1, dadurch gekennzeichnet, dass der Mantel (2) aus einem homogenen Material besteht.

3. Schaft nach Anspruch 1, dadurch gekennzeichnet, dass der Mantel (2) aus einem Verbundmaterial besteht.

4. Schaft nach einem der Ansprüche 1 - 3, dadurch gekennzeichnet, dass der Zusammenhalt zwischen Mantel (2) und Oberflächenschicht (3) kraft- und/oder formschlüssig ist.

5. Schaft nach einem der Ansprüche 1 - 4, dadurch gekennzeichnet, dass die Oberflächenschicht (3) eine Dicke von weniger als 200 µm, vorzugsweise weniger als 100 µm aufweist.

6. Schaft nach einem der Ansprüche 1 - 3, dadurch gekennzeichnet, dass die Oberflächenschicht (3) ein Verbundaufbau ist, wobei die Matrix des Verbundaufbaus vorzugsweise aus dem gleichen Material besteht, wie dasjenige des Mantels (2).

7. Schaft nach Anspruch 6, dadurch gekennzeichnet, dass die Oberflächenschicht (3) eine Dicke von weniger als 400 µm, vorzugsweise weniger als 200 µm aufweist.

8. Schaft nach einem der Ansprüche 1 - 7, dadurch gekennzeichnet, dass das Verhältnis der Volumina des Kerns (1) und Mantels (2) Vₖ/Vₘ im Bereich von 0,15 - 0,60, vorzugsweise zwischen 0,25 - 0,4 liegt.

9. Schaft nach einem der Ansprüche 1 - 8, dadurch gekennzeichnet, dass das Verhältnis der Volumina des Kerns (1) und Mantels (2) im distalen Bereich des Schaftes, welcher 2/3 der Gesamtlänge des Schaftes beträgt im Bereich von 0,10 - 0,45, vorzugsweise zwischen 0,2 - 0,3 liegt.

10. Schaft nach einem der Ansprüche 1 - 9, dadurch gekennzeichnet, dass der Kern (1) aus Reintitan oder einer Titanlegierung besteht, insbesondere aus TiAl₆V₄, TiAl₅Fe_{2.5} oder TiAl₆Nb₇.

11. Schaft nach einem der Ansprüche 1 bis 10, dadurch gekennzeichnet, dass der Mantel (2) aus einem thermoplastischen Kunststoff besteht.

12. Schaft nach Anspruch 11, dadurch gekennzeichnet, dass der thermoplastische Kunststoff Polyoxymethylen (POM), Polyether-Etherketon (PEEK), Polyaryletherketon (PAEK), Polyetherimid (PEI) oder flüssigkristallines Polymer (LCP) ist.

13. Schaft nach Anspruch 11, dadurch gekennzeichnet, dass der thermoplastische Kunststoff Polymethylpenten (PMP), Polysulfon (PSU), Polyäthersulfon (PESU oder PES), Polyäthylenterephthalat (PETP), Polymethylmethacrylat (PMMA), oder Ultrahochmolekulares-Polyäthylen (UHMW-PE) ist.

14. Schaft nach einem der Ansprüche 1 - 13, dadurch gekennzeichnet, dass die Makrostrukturierung im proximalen Bereich des Schaftes eine Rauhtiefe zwischen 0,5 - 1,5 mm aufweist.

15. Schaft nach einem der Ansprüche 1 bis 14, dadurch gekennzeichnet, dass die Mikrostrukturierung im distalen Bereich des Schaftes eine Rauhtiefe zwischen 5 - 80 µm aufweist.

16. Schaft nach einem der Ansprüche 1 - 15, dadurch gekennzeichnet, dass im proximalen Bereich des Schaftes zusätzlich zur Makrostrukturierung eine Mikrostrukturierung mit einer Rauhtiefe zwischen 0,1 - 500,0 µm, vorzugsweise zwischen 5 und 300 µm vorhanden ist.

17. Schaft nach Anspruch 16, dadurch gekennzeichnet, dass die Rauhtiefe zwischen 5 - 100 µm liegt.

18. Schaft nach Anspruch 16, dadurch gekennzeichnet, dass die Rauhtiefe zwischen 150 - 300 µm liegt.

19. Schaft nach einem der Ansprüche 1 bis 18, dadurch gekennzeichnet, dass die Oberflächenschicht (3) aus Reintitan, einer Titanlegierung, einem Bioglas, einem Calciumphosphat, insbesondere Hydroxylapatit, oder Kombinationen davon besteht.

20. Schaft nach einem der Ansprüche 1 bis 18, dadurch gekennzeichnet, dass die Oberflächenschicht (3) ein Verbundaufbau ist, wobei die Matrix dieses Verbundaufbaus vorzugsweise aus dem gleichen Material besteht, wie dasjenige des Mantels (2) und die darin eingelagerten Partikel aus Reintitan, einer Titanlegierung, einem Bioglas, einem Calciumphosphat, insbesondere Hydroxylapatit, oder Kombinationen davon bestehen.

21. Schaft nach einem der Ansprüche 1 bis 20, dadurch gekennzeichnet, dass die Oberfläche des Kernes (1) und/oder des Mantels (2) mit dreidimensionalen Strukturen (4), vorzugsweise Löchern, Rillen oder Kerben versehen ist.

22. Schaft nach einem der Ansprüche 1 bis 21, dadurch gekennzeichnet, dass der Kern (1) durchgehende Öffnungen, aufweist.

23. Schaft nach einem der Ansprüche 1 bis 22, dadurch gekennzeichnet, dass er mit einem Kragen (11) versehen ist.

24. Schaft nach einem der Ansprüche 1 bis 23, dadurch gekennzeichnet, dass er im proximo-lateralen Bereich (5) eine Bohrung (6), vorzugsweise mit einem Innengewinde (7), zur Aufnahme einer in der äusseren Kortikalis (8) zu verankernden Schraube (9) aufweist

25. Schaft nach einem der Ansprüche 1 bis 24, dadurch gekennzeichnet, dass der Kern (1) bezüglich des ihn umgebenden Mantels (2) exzentrisch angeordnet ist.

26. Schaft nach einem der Ansprüche 6 bis 25, dadurch gekennzeichnet, dass die einzelnen Partikel im Verbundaufbau der Oberflächenschicht (3) nicht direkt untereinander verbunden sind.

27. Gelenkendoprothesenkomponente für eine Gelenkendoprothese mit einem Schaft nach einem der Ansprüche 1 - 26, gekennzeichnet durch eine fest oder lösbar an der Halspartie (10) anschliessende Gelenkkugel (12).

## Claims

1. Shank for a joint-endoprosthesis comprising a metal core (1) and a proximally adjoining neck portion (10), further a non-metallic, elastic sheath (2) distally enclosing the core (1), the core (1) and the sheath (2) being held together in geometrically and/or frictionally locking manner, whereby said sheath (2) is provided with an additional surface layer (3) made of a bio-compatible material and said surface layer (3) has a higher surface hardness than that of the sheath (2),
**characterized in that**
a) the non-metallic material of the sheath (2) evinces a Young's modulus between 500 and 10'000 N/mm²;
b) the thickness of the surface layer (3) is less than 600 µm;
c) the surface layer (3) is free of inner pores;
d) the surface layer (3) comprises a macro-structure with a roughness depth of 0,2 to 2,5 mm at its outside in the proximal shank portion amounting to one-third the total shank length; and
e) the surface layer (3) comprises a micro-structure with a roughness depth between 0,1 and 500,0 µm at its outside in the distal shank portion amounting to two-thirds of the total shaft length.

2. Shank according to claim 1, characterized in that the material of the sheath (2) is homogeneous.

3. Shank according to claim 1, characterized in that the material of the sheath (2) is in compound form.

4. Shank according to one of the claims 1 to 3, characterized in that the sheath (2) and the surface layer (3) are held together in geometrically and/or frictionally locking manner.

5. Shank according to one of the claims 1 to 4, characterized in that the thickness of the surface layer (3) is less than 200 µm, preferably less than 100 µm.

6. Shank according to one of claims 1 to 3, characterized in that the surface layer (3) is a compound layer, the matrix of the compound layer preferably consisting of the material as the sheath (2).

7. Shank according to claim 6, characterized in that the thickness of the surface layer (3) is less than 400 µm, preferably less than 200 µm.

8. Shank according to one of the claims 1 to 7, characterized in that the ratio of the volumes of the core (1) to the sheath (2), namely Vₖ/Vₘ is in the range of 0,15 to 0,60, preferably between 0,25 and 0,40.

9. Shank according to one of the claims 1 to 8, characterized in that the volume-ratio of the core (1) to that of the sheath (2) is in the range of 0,10 to 0,45, preferably between 0,2 and 0,3 in the distal shank portion covering two-thirds of the total shank length.

10. Shank according to one of the claims 1 to 9, characterized in that the core (1) consists of pure titanium, or of a titanium alloy, in particular TiAl₆V₄, TiAl₅Fe_{2.5} or TiAl₆Nb₇.

11. Shank according to one of the claims 1 to 10, characterized in that the sheath (2) is a thermoplastic.

12. Shank according to claim 11, characterized in that the thermoplastic is polyoxymethylene (POM), polyether-etherketone (PEEK), polyaryl-etherketone (PAEK), polyetherimide (PEI) or a liquid-crystal polymer (LCP).

13. Shank according to claim 11, characterized in that the thermoplastic is polymethylpentene (PMP), polysulfone (PSU), polyethersulfone (PESO or PES), polyethylene terephthalate (PETP), polymethylmethacrylate (PMMA) or ultrahigh molecular-weight polyethylene (UHMW-PE).

14. Shank according to one of the claims 1 to 13, characterized in that the macro-structure in the proximal shank portion has a roughness depth between 0,5 and 1,5 mm.

15. Shank according to , characterized in that the micro-structure in the distal shank portion has a roughness depth between 5 and 80 µm.

16. Shank according to one of the claims 1 to 15, characterized in that a micro-structure with a roughness depth of 0,1 to 500,0 µm, preferably between 5 and 300 µm is present in addition to the macro-structure in the proximal shank portion.

17. Shank according to claim 16, characterized in that the depth of roughness is between 5 and 100 µm.

18. Shank according to claim 16, characterized in that the depth of roughness is between 150 and 300 µm.

19. Shank according to one of the claims 1 to 18, characterized in that the surface layer (3) consists of pure titanium, of a titanium alloy, a biological glass, a calcium phosphate, in particular hydroxyl apatite, or of a combination of these.

20. Shank according to one of the claims 1 to 18, characterized in that the surface layer (3) is a compound layer of which the matrix preferably is made of the same material as the sheath (2) and wherein the imbedded particles consist of pure titanium, a titanium alloy, a biological glass, a calcium phosphate, in particular hydroxyl apatite, or combinations thereof.

21. Shank according to one of the claims 1 to 20, characterized in that the surface of the core (1) and/or of the sheath (2) comprises three-dimensional structures (4) preferably in the form of holes, channels or notches.

22. Shank according to one of the claims 1 to 21, characterized in that through-apertures are present in the core (1).

23. Shank according to one of the claims 1 to 22, characterized by comprising a collar (11).

24. Shank according to one of the claims 1 to 23, characterized in that a borehole (6), preferably fitted with an inside thread (7) to receive a screw (9) to be anchored in the outer cortex (8), is present in its proximo-lateral portion (5).

25. Shank according to one of the claims 1 to 24, characterized in that the core (1) is mounted eccentrically relative to the sheath (2) surrounding it.

26. Shank according to one of the claims 6 to 25, characterized in that the individual particles in the compound design of the surface layer (3) are not directly connected to each other.

27. A joint-endoprosthesis component for a joint-endoprosthesis with a shank according to in one of the claims 1 to 26, characterized by a swivel ball (12) mounted in fixed or detachable manner to the neck portion (10).

## Revendications

1. Tige pour une endoprothèse de la hanche, comportant une âme métallique (1) et une partie de col (10) se raccordant à l'âme du côté proximal, ainsi qu'une enveloppe (2) entourant l'âme (1) dans la direction distale, en un matériau élastique non métallique, la cohésion entre l'âme (1) et l'enveloppe (2) s'effectuant par correspondance mécanique et/ou géométrique, l'enveloppe (2) étant dotée d'une couche superficielle (3) supplémentaire en matériau biocompatible, et la couche superficielle (3) présentant une dureté superficielle supérieure à celle de l'enveloppe (2), caractérisée en ce que
a) le matériau non métallique de l'enveloppe (2) présente un module d'élasticité de 500 et 10000 N/mm²;
b) la couche superficielle (3) présente une épaisseur inférieure à 600 µm;
c) la couche superficielle (3) est configurée sans porosité interne;
d) sur son côté extérieur, la couche superficielle (3) présente une macrostructuration dont la rugosité est d'une profondeur de 0,2 à 2,5 mm, dans la région proximale de la tige, qui vaut 1/3 de la longueur totale de la tige; et
e) sur son côté extérieur, la couche superficielle (3) présente une microstructuration avec une rugosité d'une profondeur de 0,1 à 500,0 µm, dans la région distale de la tige, qui vaut 2/3 de la longueur totale de la tige.

2. Tige selon la revendication 1, caractérisée en ce que l'enveloppe (2) est constituée d'un matériau homogène.

3. Tige selon la revendication 1, caractérisée en ce que l'enveloppe (2) est constituée d'un matériau composite.

4. Tige selon l'une des revendications 1 à 3, caractérisée en ce que la cohésion entre l'enveloppe (2) et la couche superficielle (3) est réalisée par correspondance mécanique et/ou correspondance géométrique.

5. Tige selon l'une des revendications 1 à 4, caractérisée en ce que la couche superficielle (3) présente une épaisseur inférieure à 200 µm, de préférence inférieure à 100 µm.

6. Tige selon l'une des revendications 1 à 3, caractérisée en ce que la couche superficielle (3) est une couche composite, la matrice de la structure composite étant de préférence constituée du même matériau que l'enveloppe (2).

7. Tige selon la revendication 6, caractérisée en ce que la couche superficielle (3) présente une épaisseur inférieure à 400 µm, de préférence inférieure à 200 µm.

8. Tige selon l'une des revendications 1 à 7, caractérisée en ce que le rapport des volumes de l'âme (1) et de l'enveloppe (2), Vₖ/Vₘ, est dans l'intervalle de 0,15 à 0,60, de préférence de 0,25 à 0,4.

9. Tige selon l'une des revendications 1 à 8, caractérisée en ce que le rapport des volumes de l'âme (1) et de l'enveloppe (2) dans la région distale de la tige, qui vaut 2/3 de la longueur totale de la tige, est dans l'intervalle de 0,10 à 0,45, de préférence de 0,2 à 0,3.

10. Tige selon l'une des revendications 1 à 9, caractérisée en ce que l'âme (1) est constituée de titane pur ou d'un alliage de titane, en particulier TiAl₆V₄, TiAl₅Fe_{2,5} ou TiAl₆Nb₇.

11. Tige selon l'une des revendications 1 à 10, caractérisée en ce que l'enveloppe (2) est constituée d'un matériau synthétique thermoplastique.

12. Tige selon la revendication 11, caractérisée en ce que le matériau synthétique thermoplastique est le polyoxyméthylène (POM), la polyétheréthercétone (PEEC), la polyaryléthercétone (PAEC), le polyétherimide (PEI) ou un polymère à cristaux liquides (PCL).

13. Tige selon la revendication 11, caractérisée en ce que le matériau synthétique thermoplastique est le polyméthylpentène (PMP), la polysulfone (PSU), la polyéthersulfone (PESU ou PES), le poly (téréphtalate d'éthylène) (PETP), le poly(méthacrylate de méthyle) (PMMA) ou un polyéthylène à poids moléculaire ultra élevé (PE-PMUE).

14. Tige selon l'une des revendications 1 à 13, caractérisée en ce que la macrostructuration de la région proximale de la tige présente une rugosité d'une profondeur de 0,5 à 1,5 mm.

15. Tige selon l'une des revendications 1 à 14, caractérisée en ce que la microstructuration de la région distale de la tige présente une rugosité d'une profondeur de 5 à 80 µm.

16. Tige selon l'une des revendications 1 à 15, caractérisée en ce que dans la région proximale de la tige, en plus de la macrostructuration, il existe une microstructuration avec une rugosité d'une profondeur de 0,1 à 500,0 µm, de préférence de 5 à 300 µm.

17. Tige selon la revendication 16, caractérisée en ce que la rugosité présente une profondeur de 5 à 100 µm.

18. Tige selon la revendication 16, caractérisée en ce que la rugosité est d'une profondeur de 150 à 300 µm.

19. Tige selon l'une des revendications 1 à 18, caractérisée en ce que la couche superficielle (3) est constituée de titane pur, d'un alliage de titane, d'un bioverre, d'un phosphate de calcium, en particulier l'hydroxyapatite, ou de combinaisons de ceux-ci.

20. Tige selon l'une des revendications 1 à 18, caractérisée en ce que la couche superficielle (3) est une couche composite dont la matrice est de préférence constituée du même matériau que l'enveloppe (2), et les particules qui y sont incorporées sont constituées de titane pur, d'un alliage de titane, d'un bioverre, d'un phosphate de calcium, en particulier l'hydroxyapatite, ou de combinaisons de ceux-ci.

21. Tige selon l'une des revendications 1 à 20, caractérisée en ce que la surface de l'âme (1) et/ou de l'enveloppe (2) est dotée de structures tridimensionnelles (4), de préférence des trous, des rainures ou des cannelures.

22. Tige selon l'une des revendications 1 à 21, caractérisée en ce que l'âme (1) comporte des perforations.

23. Tige selon l'une des revendications 1 à 22, caractérisée en ce qu'elle est dotée d'un collet (11).

24. Tige selon l'une des revendications 1 à 23, caractérisée en ce que dans la région proximo-latérale (5), elle présente un alésage (6) doté de préférence d'un filet intérieur (7), pour la réception d'une vis (9) à ancrer dans le cortex extérieur (8).

25. Tige selon l'une des revendications 1 à 24, caractérisée en ce que l'âme (1) est disposée en position décentrée par rapport à l'enveloppe (2) qui l'entoure.

26. Tige selon l'une des revendications 6 à 25, caractérisée en ce que les particules individuelles ne sont pas reliées directement les unes aux autres dans la structure composite de la couche superficielle (3).

27. Composant d'endoprothèse d'articulation pour une endoprothèse d'articulation présentant une tige selon l'une des revendications 1 à 26, caractérisé par une sphère d'articulation (12) se raccordant fixement ou de manière libérable à la partie de col (10).
